Europäisches Patentamt

European Patent Office

Office européen des brevets

(19)

(11) Numéro de publication : **0 022 418**
**B1**

(12) **FASCICULE DE BREVET EUROPÉEN**

(45) Date de publication du fascicule du brevet :
**11.05.83**

(51) Int. Cl.³ : **C 07 D519/02**

(21) Numéro de dépôt : **80401033.8**

(22) Date de dépôt : **09.07.80**

(54) **Procédé de préparation de l'ergotamine et des alcaloïdes du groupe ergotoxine.**

(30) Priorité : **10.07.79 FR 7917860**

(43) Date de publication de la demande :
**14.01.81 Bulletin 81/02**

(45) Mention de la délivrance du brevet :
**11.05.83 Bulletin 83/19**

(84) Etats contractants désignés :
**AT BE CH DE GB IT LI LU NL SE**

(56) Documents cités :
**DE A 697 760**
**FR A 2 089 081**

(73) Titulaire : **PIERRE FABRE S.A.**
**125, rue de la Faisanderie**
**F-75116 Paris (FR)**

(72) Inventeur : **Hatinguais, Philippe**
**"Le Landou" Chemin de Bel Air**
**F-81100 Castres (FR)**
Inventeur : **Tarroux, Roger**
**122, rue Théron Périer**
**F-81100 Castres (FR)**

(74) Mandataire : **Corre, Jacques Denis Paul et al**
**Cabinet Regimbeau 26, Avenue Kléber**
**F-75116 Paris (FR)**

Jouve, 18, rue St-Denis, 75001 Paris, France

Procédé de préparation de l'ergotamine et des alcaloïdes du groupe ergotoxine

La présente invention réalisée au Centre de Recherches Pierre Fabre a pour objet un nouveau procédé industriel d'isolement des alcaloïdes de l'ergot de seigle, en particulier de l'ergotamine et des alcaloïdes du groupe ergotoxine, qui sont des alcaloïdes utiles en thérapeutique humaine.

Plusieurs procédés ont été décrits pour l'extraction des alcaloïdes de l'ergot de seigle, mais de façon générale ils se sont révélés inapplicables à une exploitation industrielle en raison de la grande instabilité des alcaloïdes et des faibles rendements obtenus.

L'extraction est généralement réalisée à partir de l'ergot de seigle pulvérisé, préalablement dégraissé à l'aide d'éther de pétrole ou d'hexane. Les solvants les plus couramment utilisés sont l'alcool, les mélanges hydroalcooliques (SANDOZ, 1929, DE-A-47 315) l'acétone (ARZNEI-MITTEL WERKE, 1962, DE-A-1 240 871) (RICHTER GEDEON, 1966, HU-A-152 934) le méthanol aqueux (L. VOGEL, DE-A-697 760). Les extractions sont réalisées en milieu alcalin, par addition de carbonate ou bicarbonate de sodium, ou d'ammoniaque. Plus rares sont celles effectuées en milieu acide : trichloréthylène et acide acétique (KUCZYNSKI, 1957, PL-A-40 884) méthanol et acide tartrique (L. VOGEL, 1937, DE-A-697 760).

L'extraction sans dégraissage préalable est réalisée avec un solvant non miscible à l'eau tel que le chloroforme, le benzène, le trichloréthylène, le toluène, le chlorure de méthylène ou le dichloréthane (LEK, 1971, FR-A-2 089 081).

A partir de ce premier extrait les alcaloïdes sont obtenus par concentration et extraction liquide-liquide par une solution acide (JOHN WYETH and BROTHER, Incorporated, 1935, US-A-2 120 635) par fixation sur des résines cationiques (KUCZYNSKI, 1957, PL-A-40 884) ou sur colonne d'Alumine (LEK, 1971, FR-A-2 089 081). Dans ce dernier cas, l'élution de l'ensemble des alcaloïdes est réalisée par addition de méthanol ou d'éthanol sans qu'il y ait séparation individuelle de ceux-ci.

La plupart des procédés décrits antérieurement ne tiennent pas compte de la facilité de décomposition et de transformation en isomères inactifs. L'ergotamine est particulièrement fragile en milieu alcalin, ce qui proscrit toutes les extractions initiales en milieu sodique ou ammoniacal. En outre, les opérations décrites entraînent, à l'échelle industrielle, des durées incompatibles avec l'instabilité des alcaloïdes. D'autre part, les techniques antérieures décrivent le plus souvent l'isolement d'un seul alcaloïde, ce qui ne présente aucun intérêt sur le plan économique. Il faut également souligner que toutes les techniques mettant en œuvre des extractions liquide-liquide (par exemple CS-A-113 109), qui sont particulièrement délicates à mettre en œuvre lorsque les alcaloïdes sont impurs en raison d'émulsions très stables. La mise en œuvre de résines cationiques (PL-A-40 884) nécessite des débits très faibles, de ce fait l'exploitation industrielle conduit à des dégradations importantes.

Les très nombreuses publications relatives à la préparation des alcaloïdes de l'ergot de seigle ne peuvent être mises en œuvre industriellement en vue de l'obtention d'ergotamine et des autres alcaloïdes du groupe ergotoxine avec des rendements acceptables du point de vue industriel.

La présente invention avait précisément pour but de remédier à un tel inconvénient majeur.

Conformément au procédé de la présente invention, on réalise les opérations successives suivantes :

a) préparation des sclérotes par broyage de l'ergot de seigle (Claviceps purpurea), préalablement imprégné d'eau ou d'une solution aqueuse alcaline, par écrasement, à une granulométrie inférieure à 0,5 mm ;

b) extraction en milieu acide du totum d'alcaloïdes à l'aide d'acétate d'éthyle, suivie de l'isolement du totum d'alcaloïdes par précipitation à l'aide d'hydrocarbures saturés en $C_6$ ou $C_7$ ;

c) purification des alcaloïdes obtenus en b) par solubilisation dans l'acétate d'éthyle et extraction par une solution aqueuse acide puis précipitation des alcaloïdes ;

d) séparation chromatographique de l'ergotamine des autres alcaloïdes sur alumine acide et élution par la méthyléthylcétone contenant 1 % d'eau.

En outre, le procédé de l'invention offre la possibilité de recycler les solvants et l'alumine acide, de stocker intermédiairement les alcaloïdes et d'introduire les isomères dextrogyres à l'étape b) après avoir procédé à une rétroisomérisation.

D'autres caractéristiques et avantages du procédé de l'invention apparaîtront à la lecture de la description détaillée faite ci-après, notamment à propos d'un exemple particulier de mise en œuvre.

Il a tout d'abord été démontré que la granulométrie de l'ergot de seigle broyé était particulièrement importante, et que des particules de dimensions supérieures à 0,5 mm ne sont que très difficiles à épuiser, et ceci même après une macération de plusieurs heures.

Il a par ailleurs été constaté que la vitesse d'extraction se trouvait considérablement améliorée lorsque les sclérotes étaient soumis à un écrasement dans un appareil dont la distance entre les pièces mobiles est de l'ordre de 0,2-0,5 mm.

Selon le procédé de l'invention les sclérotes sont au préalable imprégnés d'eau de façon à rendre la matière plus molle et à faciliter son écrasement.

De façon surprenante, le rendement d'extraction a ainsi pu être augmenté de 10 à 20 % comparativement aux techniques antérieures connues. Il convient enfin de noter que l'eau peut être remplacée par une solution alcaline, ce qui permet d'assurer simultanément une dépigmentation.

Au cours de l'étape b), le totum des alcaloïdes peut être avantageusement obtenu, sans dégraissage préalable, au moyen d'un solvant miscible ou partiellement miscible avec l'eau. Bien que les cétones ou les alcools conduisent à des résultats convenables, l'utilisation de l'acétate d'éthyle a été préférée. Dans tous les cas, le solvant utilisé a été additionné d'une solution aqueuse d'acide minéral ou organique, ce qui permet une bonne stabilisation chimique des alcaloïdes. Parmi les acides les plus avantageux on citera à titre d'exemple l'acide chlorhydrique, sulfurique, phosphorique, citrique et plus spécialement l'acide tartrique, sans que la teneur exacte en eau ou en acide soit limitative. Selon un mode de mise en œuvre préféré du procédé de l'invention, le solvant organique contiendra sensiblement de 5 à 10 % d'une solution aqueuse contenant d'environ 1 à environ 5 % d'acide.

Après concentration, le solvant organique contenant les sels d'alcaloïdes est précipité par addition d'hydrocarbures saturés en $C_6$ ou $C_7$. Selon un mode de précipitation préféré, les sels d'alcaloïdes sont obtenus par préconcentration de l'extrait organique, puis addition d'heptane et évaporation du solvant d'extraction résiduel. Ce procédé permet l'évaporation du solvant d'extraction sans l'intermédiaire de l'extrait mou, difficile à manipuler sans perte à l'échelle industrielle.

Les alcaloïdes bruts ainsi obtenus se présentent sous forme d'une poudre brune, qui peut éventuellement être conservée plusieurs mois sans la moindre altération.

L'étape c) du procédé de l'invention consiste en une purification des alcaloïdes totaux. Les alcaloïdes obtenus à l'issue de l'étape b) sont solubilisés dans l'acétate d'éthyle, duquel les alcaloïdes sont extraits par une solution aqueuse acide. Parmi les acides utilisés, on mentionnera à titre d'exemple les acides chlorhydrique, sulfurique, phosphorique, citrique et plus particulièrement tartrique.

La phase aqueuse est neutralisée et saturée à l'aide d'un sel minéral, ce qui provoque la précipitation des alcaloïdes.

La dernière étape d) du procédé objet de la présente invention est une chromatographie. Les alcaloïdes obtenus à l'étape c) sont solubilisés dans l'acétone anhydre et la solution obtenue peut être avantageusement filtrée sur alumine basique, ce qui provoque l'élimination des pigments subsistant après l'étape précédente. La solution acétonique incolore est alors concentrée à un volume minimum de l'ordre de 25 ml/kg d'ergot de seigle de départ, et chargée sur une colonne chromatographique d'alumine acide. L'élution est réalisée par la méthyl-éthyl-cétone contenant 1 % d'eau.

Le déroulement de la chromatographie est suivi à l'aide d'un détecteur, suivant les techniques connues.

Pareille séparation conduit à une première fraction chromatographique (A) qui contient les isomères dextrogyres et par exemple l'ergocristine, ainsi qu'à une seconde fraction chromatographique (B), qui contient l'ergotamine. La fraction (A) est concentrée, titrée et additionnée de la quantité d'acide phosphorique nécessaire à la salification de l'ergocristine. Le précipité de phosphate d'ergocristine est isolé par filtration lavé à l'acétone et séché sous vide à 60 °C. Le filtrat est traité suivant une technique connue pour subir une rétro-isomérisation. Il sera ensuite recyclé à l'étape c). Les alcaloïdes dextrogyres subissant la rétro-isomérisation sont principalement l'ergotaminine, l'ergocristinine, l'ergocryptinine et l'ergocorninine.

La fraction (B) est alors concentrée et l'ergotamine base est cristallisée sous forme de sel suivant le même principe que ci-dessus ou sous forme de base par addition éventuelle d'un non solvant du type éther de pétrole, hexane, et de préférence à l'aide d'heptane, après reprise de l'acétone.

Les alcaloïdes obtenus avec un rendement global supérieur à 70 % ont un degré de pureté de l'ordre de 98 % et sont susceptibles d'être hydrogénés en présence de catalyseurs dans des solvants tels que le dioxanne, le tétrahydrofuranne ou le diméthylformamide selon des techniques connues.

Le procédé objet de la présente invention peut également être applicable à la séparation de mélanges ergotamine-ergocornine, ergotamine-ergocryptine, ou ergotamine-ergotoxine, suivant la composition particulière des ergots de seigle utilisés.

Exemple particulier de mise en œuvre du procédé de l'invention

1 kg de végétal (représentant 4,0 g d'alcaloïdes) finement broyé est additionné de 0,3 litre d'acide tartrique à 2,5 % et épuisé à température ambiante par trois fois 4 litres d'acétate d'éthyle saturé d'eau. Les jus d'extraction sont concentrés à 500 ml, additionnés d'un litre d'heptane et reconcentrés à 500 ml. Les alcaloïdes totaux ainsi précipités sont repris par 1 litre d'acétate d'éthyle saturé d'eau puis extraits par trois fois 1 litre d'acide tartrique à 2,5 %. Les alcaloïdes bases sont précipités de la phase aqueuse par neutralisation et addition de 300 g de chlorure de sodium.

Le précipité alcaloïdique obtenu (3,4 g) séché est repris à l'acétone (400 ml) et filtré sur une colonne contenant 12 g d'alumine basique. Les jus acétoniques, concentrés à 25 ml sont ensuite chromatographiés sur une colonne de 120 g d'alumine acide.

600 ml de méthyl-éthyl-cétone à 1 % d'eau éluent une fraction dans laquelle on précipite, après dosage, le phosphate d'ergocristine par addition stoechiométrique d'acide phosphorique.

150 ml de méthanol éluent une fraction qui est reprise sur 10 ml d'acétone et l'addition de 1 ml d'heptane précipite l'ergotamine base.

A partir des alcaloïdes ainsi obtenus, on peut préparer, selon les méthodes habituelles, les sels d'alcaloïdes conformes aux critères de pureté pharmaceutique.

**Revendications**

1. Procédé de préparation de l'ergotamine et des alcaloïdes du groupe ergotoxine caractérisé en ce qu'on réalise les opérations successives suivantes :

a) préparation des sclérotes par broyage de l'ergot de seigle (Claviceps purpurea), préalablement imprégné d'eau ou d'une solution aqueuse alcaline, par écrasement, à une granulométrie inférieure à 0,5 mm ;

b) extraction en milieu acide du totum d'alcaloïdes à l'aide d'acétate d'éthyle, suivie de l'isolement du totum d'alcaloïdes par précipitation à l'aide d'hydrocarbures saturés en $C_6$ ou $C_7$ ;

c) purification des alcaloïdes obtenus en b) par solubilisation dans l'acétate d'éthyle et extraction par une solution aqueuse acide puis précipitation des alcaloïdes ;

d) séparation chromatographique de l'ergotamine des autres alcaloïdes sur alumine acide et élution par la méthyléthylcétone contenant 1 % d'eau.

2. Procédé selon la revendication 1, caractérisé en ce que l'on ajoute au solvant d'extraction utilisé au cours de l'étape b) de 1 à 10 % d'une solution aqueuse contenant 1 à 5 % d'acide.

3. Procédé selon l'une des revendications 1 et 2, caractérisé en ce que ledit acide est l'acide tartrique.

4. Procédé selon l'une des revendications 1 à 3, caractérisé en ce que les alcaloïdes lévogyres sont précipités de la première fraction chromatographique par addition d'acide phosphorique.

5. Procédé selon l'une des revendications 1 à 3, caractérisé en ce que l'ergotamine est cristallisée de la seconde fraction chromatographique sous forme de base ou sous forme de sel en particulier de tartrate.

6. Procédé selon l'une des revendications 1 à 5, selon lequel les alcaloïdes en solution dans l'acétone subissent, avant chromatographie, une filtration sur alumine basique pour retenir les pigments.

7. Procédé selon l'une des revendications 1 à 6, caractérisé en ce que la première fraction chromatographique, après isolement des alcaloïdes lévogyres, est soumise à une opération de rétro-isomérisation en vue d'un recyclage en amont de la chromatographie.

**Claims**

1. A process for the preparation of ergotamine and alkaloids of the ergotoxine group, characterised in that the following operations are carried out in succession :

a) preparation of sclerotes by grinding ergot of rye (Claviceps purpurea), previously impregnated with water or with an aqueous alkaline solution, by crushing to a grain size of less than 0.5 mm ;

b) extraction in an acid medium of all the alkaloids using ethyl acetate, followed by isolation of all the alkaloids by precipitation using $C_6$ or $C_7$ saturated hydrocarbons ;

c) purification of the alkaloids obtained in b) by solubilization in ethyl acetate and extraction using an aqueous acid solution, then precipitation of the alkaloids ;

d) chromatographic separation of ergotamine from the other alkaloids on acid alumina and elution using methylethylketone containing 1 % of water.

2. A process according to claim 1, characterised in that from 1 to 10 % of an aqueous solution containing from 1 to 5 % acid is added to the extraction solvent which is used during stage b).

3. A process according to one of claims 1 and 2, characterised in that said acid is tartaric acid.

4. A process according to one of claims 1 to 3, characterised in that levorotatory alkaloids are precipitated from the first chromatographic fraction by the addition of phosphoric acid.

5. A process according to one of claims 1 to 3, characterised in that the ergotamine is crystallised from the second chromatographic fraction in the form of a base or in the form of a salt, in particular in the form of the tartrate.

6. A process according to one of claims 1 to 5, according to which the alkaloids in solution in acetone undergo, before chromatography, a filtration on basic alumina to retain pigments.

7. A process according to one of claims 1 to 6, characterised in that, after isolation of the levorotatory alkaloids, the first chromatographic fraction is subjected to a retro-isomerisation operation for the purpose of recycling upstream of the chromatography.

**Ansprüche**

1. Verfahren zur Herstellung von Ergotamin und von Alkaloiden der Gruppe Ergotoxin, dadurch gekennzeichnet, daß man die folgenden Schritte aufeinanderfolgend durchführt :

a) Herstellung von Sklerotien durch Zerreiben von Mutterkorn (Claviceps purpurea), das vorher mit Wasser oder einer wässerigen alkalischen Lösung getränkt wurde, durch Zermalmen auf eine Korngröße unterhalb 0,5 mm ;

b) Extraktion der Gesamtalkaloide mit Hilfe von Äthylacetat in saurem Milieu und nachfolgende Isolierung der Gesamtalkaloide durch Ausfällung mit Hilfe von gesättigten $C_6$- oder $C_7$-Kohlenwasserstoffen ;

c) Reinigung der in b) erhaltenen Alkaloide durch Auflösen in Äthylacetat und Extraktion mit einer wässerigen sauren Lösung und nachfolgende Ausfällung der Alkaloide ;

d) chromatographische Abtrennung des Ergotatmins von den anderen Alkaloiden auf saurer Tonerde und Eluieren durch Methyläthylketon mit einem Gehalt von 1 % Wasser.

2. Verfahren gemäß Anspruch 1, dadurch gekennzeichnet, daß man dem in Stufe b) verwendeten Extraktionslösungsmittel eine 1 bis 10 %ige wässerige Lösung mit einem Säuregehalt von 1 bis 5 % zusetzt.

3. Verfahren gemäß Anspruch 1 oder 2, dadurch gekennzeichnet, daß die genannte Säure Weinsäure ist.

4. Verfahren nach einem der Ansprüche 1 bis 3, dadurch gekennzeichnet, daß die linksdrehenden Alkaloide aus der ersten chromatographischen Fraktion durch Zugabe von Phosphorsäure ausgefällt werden.

5. Verfahren gemäß einem der Ansprüche 1 bis 3, dadurch gekennzeichnet, daß das Ergotamin aus der zweiten chromatographischen Fraktion in Form der Base oder in Form des Salzes, insbesondere des Tartrats, kristallisiert wird.

6. Verfahren gemäß einem der Ansprüche 1 bis 5, gemäß welchem die Alkaloide in Lösung in Aceton vor der Chromatographie einer Filtration auf basischer Tonerde unterworfen werden, um die Pigmente zurückzuhalten.

7. Verfahren gemäß einem der Ansprüche 1 bis 6, dadurch gekennzeichnet, daß die erste chromatographische Fraktion nach der Isolierung der linksdrehenden Alkaloide einer Rückisomerisierung unterworfen wird, um der Chromatographie am Anfang wieder zugeführt zu werden.